# EUROPÄISCHE PATENTANMELDUNG

(11) **EP 4 119 173 A1**
(43) Veröffentlichungstag der Anmeldung: **18.01.2023**
(21) Anmeldenummer: 21185683.6
(22) Anmeldetag: 14.07.2021
(51) Int. Cl.: A61M 5/31, A61M 5/34

(54) **VERSCHLUSSKAPPE UND VERSCHLUSSSYSTEM FÜR EINE MIT EINEM MEDIKAMENT ODER MEDIZINISCHEN WIRKSTOFF VORBEFÜLLTE SPRITZE**

(71) Anmelder: Spichiger, Marco, 2544 Bettlach (CH)
(72) Erfinder: Spichiger, Marco, 2544 Bettlach (CH)
(74) Vertreter: Tergau & Walkenhorst Patentanwälte PartGmbB

(57) **Zusammenfassung**

Eine Verschlusskappe (2, 2') für eine mit einem Medikament oder medizinischen Wirkstoff vorbefüllte Spritze (40, 40') soll bei einfacher Handhabung zuverlässig eine hohe Dichtigkeit gewährleisten. Dazu umfasst die Verschlusskappe (2, 2') erfindungsgemäß eine starre, in einem Kontaktbereich (16) mit einem Außengewinde (18) versehenen Außenkappe (6, 6'), die einen sich in einer Längsrichtung erstreckenden Innenkanal (14) aufweist, in dem ein Dichtungsstopfen (8, 8') aus einem im Vergleich zur Außenkappe (6, 6') weicheren und besser verformbaren Material, vorzugsweise aus Gummi oder TPE, angeordnet ist.

## Beschreibung

Die Erfindung betrifft eine Verschlusskappe für einen Medikamentenbehälter, insbesondere für eine mit einem Medikament oder medizinischen Wirkstoff vorbefüllte Spritze. Sie bezieht sich weiter auf ein eine solche Verschlusskappe umfassendes Verschlusssystem.

Medikamente, insbesondere für die Behandlung in hoch spezialisierten oder komplexen Therapien, werden üblicherweise in Wirkstoff- oder Medikamentenbehältern, auch als Container oder Phiole bezeichnet, bereitgestellt. Alternativ können Medikamente aber auch in vorbefüllten Spritzen oder Spritzenkörpern bereitgestellt werden. Solche vorbefüllten Spritzen, die üblicherweise die nachfolgende Handhabung durch das Personal, insbesondere bei der Verabreichung des Wirkstoffs, deutlich erleichtern, umfassen üblicherweise den eigentlichen, mit dem Wirkstoff befüllten Spritzenkörper, an den zum Zweck der Verabreichung unmittelbar vor dem Einsatz der Spritze die Spritzennadel angebracht, beispielsweise angeschraubt, wird.

Gerade in modernen medizinischen Verfahren oder Therapien können Medikamente oder Substanzen zum Einsatz kommen, die an sich eigentlich toxisch oder auf sonstige Weise schädlich oder gefährlich sind. Für das mit der Handhabung solcher Substanzen betraute Personal, wie beispielsweise Apotheker und Krankenschwestern, können somit akute und langfristige Gesundheitsrisiken entstehen, gerade wenn es wiederholt Medikamenten oder Lösungsmitteln ausgesetzt ist, die während der Zubereitung, der Verabreichung von Medikamenten und anderen ähnlichen Behandlungen in die Luft entweichen könnten. Dieses Problem kann besonders schwerwiegend sein, wenn es sich um Zytotoxine, antivirale Medikamente, Antibiotika oder Radiopharmazeutika handelt. Die durch die Exposition gegenüber diesen Medikamenten potentiell entstehenden Gesundheitsrisiken umfassen ein erhöhtes Krebsrisiko, genetische Veränderungen und dergleichen. Des Weiteren ist es auch im Hinblick auf den Umstand, dass in jüngster Zeit Medikamente mit einem äußerst hohen Dosispreis zugelassen worden sind, dringend wünschenswert oder sogar notwendig, die unbeabsichtigte Abgabe auch kleinster Mengen solcher Medikamente oder Wirkstoffe an die Umgebung zuverlässig zu vermeiden.

Es ist daher allgemein wünschenswert, die bei der Verabreichung von Medikamenten verwendeten Systeme oder Komponenten für besonders geringe Materialoder Wirkstoffverluste während der gesamten Handhabungskette auszulegen. Für Behältnisse, in denen die Medikamente kurzfristig oder über einen gewissen Zeitraum hinweg vorgehalten werden, also insbesondere auch für vorbefüllte Spritzen oder vorbefüllte Spritzenkörper, ist somit eine hohe Dichtigkeit des Verschlusssystems wünschenswert. Darüber hinaus sollen die eingesetzten Komponenten natürlich aber auch besonders einfach und zuverlässig handhabbar und zudem kostengünstig sein.

Der Erfindung liegt daher die Aufgabe zugrunde, eine Verschlusskappe für einen Medikamentenbehälter, insbesondere für eine mit einem Medikament oder mit einem medizinischen Wirkstoff vorbefüllte Spritze oder einen mit einem Medikament oder mit einem medizinischen Wirkstoff vorbefüllten Spritzenkörper, anzugeben, mit der bei einfacher Handhabung zuverlässig eine hohe Dichtigkeit erreichbar ist. Sie bezieht sich weiter auf ein eine solche Verschlusskappe umfassendes Verschlusssystem.

Bezüglich der Verschlusskappe wird diese Aufgabe erfindungsgemäß gelöst mit einer starren, in einem Kontaktbereich zum Aufschrauben auf den Mündungsbereich der Spritze mit einem Außengewinde versehenen Außenkappe, die einen sich in einer Längsrichtung erstreckenden Innenkanal aufweist, in dem ein Dichtungsstopfen aus einem im Vergleich zur Außenkappe weicheren und besser verformbaren Material, vorzugsweise aus Gummi oder TPE, angeordnet ist.

Vorteilhafte Ausgestaltungen der Erfindung sind Gegenstand der Unteransprüche.

Die Erfindung geht von der Überlegung aus, dass die angestrebte hohe Dichtigkeit beim Verschluss des vorbefüllten Spritzenkörpers erreicht werden kann, indem ein hinsichtlich seiner Materialeigenschaften gezielt auf diese Dichtwirkung hin ausgerichtetes Dichtelement bereitgestellt wird. Dieses sollte aus einem geeigneten, einerseits mit den üblicherweise für solche Spritzenkörper verwendeten Basismaterialien wie insbesondere Glas oder Kunststoff kompatiblem Material mit geeigneten plastischen Eigenschaften bestehen. Um dabei aber auch einen ausreichenden Schutz gegenüber äußeren Einwirkungen und damit eine hohe Zuverlässigkeit bei der vorübergehenden Aufbewahrung der Wirkstoffe zu gewährleisten, sollte dieses Dichtelement nach außen hin von einer in der Art einer Schutzkappe ausgeführten Außenkappe umgeben sein. Die Verschlusskappe kann somit in der Art einer zweikomponentigen Ausgestaltung ausgeführt sein, wobei in der Art einer funktionalen Trennung die erwünschte hohe Dichtwirkung durch das in dieser Hinsicht geeignet ausgelegte Dichtelement und andererseits die mechanische Stabilität und Belastbarkeit durch die schützende Außenkappe gegeben ist. Die Außenkappe kann in dieser Ausgestaltung zudem noch für eine besonders erleichterte und damit handhabungsfreundliche Montage am Spritzenkörper ausgelegt sein, indem sie an geeigneter, für die Verbindung mit dem Spritzenkörper vorgesehener Position mit einem Außengewinde versehen ist.

Hinsichtlich der Materialwahl sind die genannten Komponenten vorteilhafterweise gezielt für die beabsichtigte Verwendung für medizinische Wirkstoffe und eine Kompatibilität mit den sonstigen in diesem Bereich üblichen Werkstoffen ausgelegt. Dazu ist die Außenkappe vorteilhafterweise aus einem Kunststoff, vorzugsweise aus Polypropylen (PP), einem Polyolefin, Cyclo-Olefin-Copolymer (COC), Cyclo-Olefine-Polymer (COP) oder Polycarbonat gefertigt. In weiterer vorteilhafter Ausgestaltung sind sowohl der Dichtungsstopfen als auch die Außenkappe jeweils aus Material der Qualitätsstufe "medical grade" gefertigt.

Im Sinne einer besonders erleichterten Handhabung ist die Verschlusskappe für eine Anbringung am und auch ein späteres Lösen vom Spritzenkörper durch Schrauben, also durch An- bzw. Abschrauben, ausgelegt; dies wird insbesondere durch das an der Außenkappe vorgesehene Außengewinde erreicht. Um dabei die An- oder Abschraubvorgänge in ihrer Handhabung für das Bedienpersonal besonders zu erleichtern, ist die Außenkappe in besonders vorteilhafter Ausgestaltung mit geeigneten Griffelementen versehen, die für den Bediener den Kraftschluss bei der Schraubbewegung besonders erleichtern. Dazu ist die Außenkappe in besonders vorteilhafter Ausgestaltung in einem in Längsrichtung gesehen versetzt zum Kontaktbereich angeordneten Handhabungsbereich an ihrem Außenumfang mit einer Anzahl von, vorzugsweise mit zwei, sich in Längsrichtung erstreckenden Griffrillen versehen.

Das Außengewinde der Außenkappe ist vorzugsweise als Luer-Gewinde ausgeführt.

Die Verschlusskappe ist ihrer Bauweise nach grundsätzlich zweikomponentig ausgeführt. Für eine erleichterte Handhabung sollten die Komponenten aber vorzugsweise vormontiert sein, so dass bei der eigentlichen Anbringung der Verschlusskappe am Spritzenkörper lediglich ein Bauteil aufgebracht werden muss. Dazu ist in besonders vorteilhafter Ausgestaltung der in der Außenkappe zur Aufnahme des Dichtungsstopfens vorgesehene Innenkanal mit einer umlaufenden Rastkante versehen. Korrespondierend dazu weist in dieser bevorzugten Ausführungsform der Dichtungsstopfen eine daran angepasste, außenseitig umlaufende Rastkante auf. Diese ist vorzugsweise derart dimensioniert und positioniert, dass der Dichtungsstopfen nach seinem Einschieben in den Innenkanal durch Verhakung der beiden Rastkanten rastend im Innenkanal der Außenkappe fixiert wird.

In alternativer, als eigenständig erfinderisch angesehener Ausgestaltung kann die Verschlusskappe aber auch durch ein Zwei-Komponenten-Spritzgussverfahren hergestellt werden, bei dem die beiden Komponenten von vornherein gemeinsam miteinander produziert werden.

Die vorstehend beschriebene Verschlusskappe mit ihren bevorzugten Ausführungsformen ist dem Grunde nach für ein Anschrauben an den eigentlichen Spritzenkörper vorgesehen. Sie kann somit unmittelbar verwendet werden, falls der Spritzenkörper nach seiner grundsätzlichen Auslegung ohnehin bereits mit einem geeigneten Gewindesystem ausgerüstet ist, beispielsweise um später einen Nadelhalter oder dergleichen am Mündungsbereich des Spritzenkörpers anschrauben zu können.

In einer anderen Bauweise können Spritzenkörper der genannten Art mit einem sich verjüngenden Mündungsbereich versehen sein, auf den bei der Nutzung der Spritze beispielsweise der Nadelhalter aufgeklemmt werden kann. Um die Verschlusskappe auch bei einem solchen, auch als "Standard-Spritze" bezeichneten, Spritzenkörper verwenden zu können, ist in als eigenständig erfinderisch angesehener Ausgestaltung ein Verschlusssystem für eine mit einem Medikament oder medizinischen Wirkstoff vorbefüllte Spritze mit einer Verschlusskappe der genannten Art vorgesehen. Das Verschlusssystem umfasst, ergänzend zur genannten Verschlusskappe, dabei auch noch ein Gewinde-Adapterstück, das in einem Außenmantel mit einem an das Außengewinde der Außenkappe angepassten Innengewinde versehen ist, und das einen auf einen Endbereich des Spritzenkörpers aufschiebbaren, mit einer Anzahl von umlaufend angeordneten Klemmfedern versehenen Klemmring aufweist. Vorteilhafterweise ist das Gewinde-Adapterstück dabei aus einem Kunststoff, vorzugsweise aus Polypropylen (PP), einem Polyolefin, Cyclo-Olefin-Copolymer (COC), Cyclo-Olefine-Polymer (COP) oder Polycarbonat gefertigt.

Die mit der Erfindung erzielten Vorteile bestehen insbesondere darin, dass durch die mehrkomponentige Ausgestaltung der Verschlusskappe eine Mehrzahl eigentlich konkurrierender Auslegungsziele, nämlich beispielsweise hohe Dichtigkeit, hohe Zuverlässigkeit, mechanische Belastbarkeit und/oder gute Handhabbarkeit, gleichermaßen erreicht werden können. Zusätzlich ist, insbesondere über die geeignete Konturierung der Außenkappe in ihrem Außenbereich, ein intuitiv nutzbarer Abdrehmechanismus gegeben. Die Verschlusskappe gewährleistet die Integrität des Behälters oder des Spritzenkörpers, kann aber dennoch leicht geöffnet werden. Sie bietet zudem eine nahtlose Passform für vorgefüllte Glas- oder Polymerspritzen. Die Integrität der Containerschließung ist allgemein ein wichtiges Anliegen in der Pharmaindustrie. Dieses wird ebenso erreicht wie die ebenfalls bedeutsame Benutzerfreundlichkeit.

Ein Ausführungsbeispiel der Erfindung wird anhand einer Zeichnung näher erläutert. Darin zeigen:
- Fig. 1: ein Verschlusssystem für einen Medikamentenbehälter, insbesondere für eine mit einem Medikament oder medizinischen Wirkstoff vorbefüllte Spritze, in Explosionsdarstellung,
- Fig. 2: eine Außenkappe der Verschlusskappe des Verschlusssystems nach Fig. 1 in zwei unterschiedlichen Längsschnitten,
- Fig. 3: einen Dichtungsstopfen der Verschlusskappe des Verschlusssystems nach Fig. 1 im Längsschnitt,
- Fig. 4: ein Gewinde-Adapterstück 4 des Verschlusssystems nach Fig. 1,
- Fig. 5: die Verschlusskappe des Verschlusssystems gem. Fig. 1 im perspektivischen Schnitt,
- Fig. 6: das Verschlusssystem nach Fig. 1 in montiertem Zustand in perspektivischer Ansicht,
- Fig. 7: das Verschlusssystem nach Fig. 1 in montiertem Zustand in zwei verschiedenen Längsschnitten,
- Fig. 8: ein alternatives Verschlusssystem für einen Medikamentenbehälter, insbesondere für eine mit einem Medikament oder medizinischen Wirkstoff vorbefüllte Spritze, in Explosionsdarstellung,
- Fig. 9: die Verschlusskappe des Verschlusssystems gem. Fig. 8 im Längsschnitt in Explosionsdarstellung,
- Fig. 10: die Verschlusskappe des Verschlusssystems gem. Fig. 8 im perspektivischen Schnitt,
- Fig. 11: das Verschlusssystem gem. Fig. 8 in Explosionsdarstellung,
- Fig. 12: das Verschlusssystem gem. Fig. 8 in perspektivischer Ansicht,
- Fig. 13: das Verschlusssystem gem. Fig. 8 im perspektivischen Schnitt,
- Fig. 14: das Verschlusssystem gem. Fig. 8 im Längsschnitt,
- Fig. 15: einen mit einer Verschlusskappe gem. Fig. 9 verschlossenen Spritzenkörper mit angeformtem Gewindesystem vor (Fig. 15a) und nach (Fig. 15b) Anbringung der Verschlusskappe,
- Fig. 16: einen mit einer Verschlusskappe gem. Fig. 5 verschlossenen Spritzenkörper mit angeformtem Gewindesystem vor (Fig. 16a) und nach (Fig. 16b) Anbringung der Verschlusskappe,
- Fig. 17: einen mit einem Verschlusssystem gem. Fig. 8 verschlossenen Spritzenkörper vor (Fig. 17a) und nach (Fig. 17b) Anbringung des Verschlusssystems, und
- Fig. 18: einen mit einem Verschlusssystem gem. Fig. 1 verschlossenen Spritzenkörper vor (Fig. 18a) und nach (Fig. 18b) Anbringung des Verschlusssystems.

Gleiche Teile sind in allen Figuren mit denselben Bezugszeichen versehen.

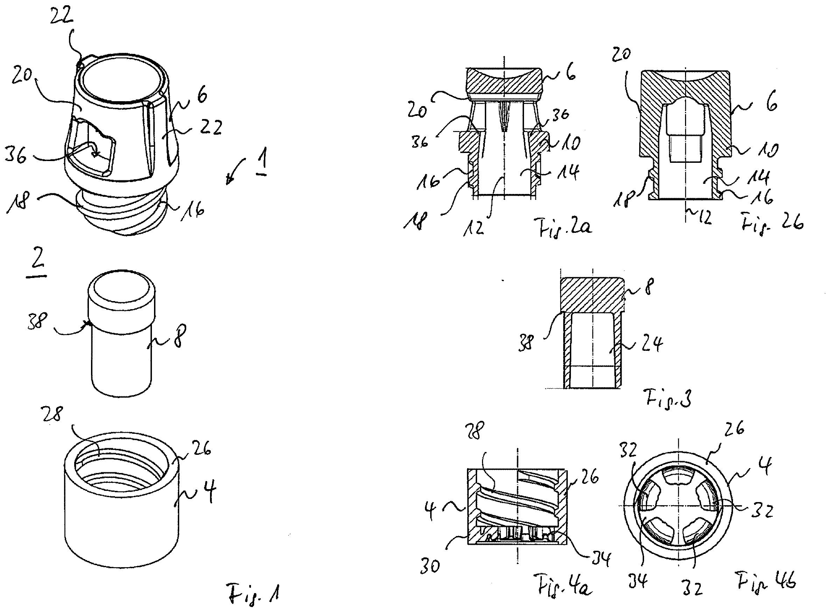

Das in Fig. 1 in Explosionsdarstellung gezeigte Verschlusssystem 1 umfasst als Hauptkomponenten die eigentlich zum Verschließen eines mit einem Medikament oder einem medizinischen Wirkstoff vorbefüllten Behälters, insbesondere einer Spritze, vorgesehene Verschlusskappe 2 sowie ein zugeordnetes Gewinde-Adapterstück 4. Die Verschlusskappe 2 ist dabei ihrerseits mehr-, insbesondere zwei-, komponentig ausgeführt, so dass in der Art einer funktionalen Trennung mehrere eigentlich konkurrierende Auslegungsziele erreicht werden können.

Dazu umfasst die Verschlusskappe 2 als wesentliche Komponenten einerseits eine starre Außenkappe 6 und andererseits einen Dichtungsstopfen 8 aus einem im Vergleich zur Außenkappe 6 weicheren und besser verformbaren Material, nämlich insbesondere aus Gummi oder TPE. Die Außenkappe 6, die in Fig. 2 in zwei verschiedenen Längsschnitten gezeigt ist, wird von einem Grundkörper 10 gebildet, der seinerseits im Ausführungsbeispiel aus einem Kunststoff, insbesondere aus Polypropylen (PP), einem Polyolefin, Cyclo-Olefin-Copolymer (COC), Cyclo-Olefine-Polymer (COP) oder Polycarbonat, jeweils insbesondere in der Qualitätsstufe "medical grade" gefertigt ist. In diesen Grundkörper 10 ist ein sich in der durch die Längsachse 12 angedeuteten Längsrichtung erstreckender Innenkanal 14 eingearbeitet, der zur Aufnahme des Dichtstopfens 8 vorgesehen ist.

Der Grundkörper 10 der Außenkappe 6 ist in einem "unteren", zur Herstellung der Verbindung mit dem Behälter oder insbesondere der vorbefüllten medizinischen Spritze vorgesehenen Kontaktbereich 16 mit einem - insbesondere im Hinblick auf gängige Verbindungssysteme im Bereich medizinischer Gerätschaften vorzugsweise als Luer-Gewinde ausgestalteten - Außengewinde 18 versehen.

Die Verschlusskappe 2 ist somit, entsprechend der Ausgestaltung der Außenkappe 6, für eine Schraubverbindung mit dem Medikamentenbehälter bzw. der vorbefüllten medizinischen Spritze vorgesehen. Um dabei eine besonders erleichterte Handhabung der Verschlusskappe 2 bei der Anbringung am und auch beim späteren Lösen vom Spritzenkörper durch Schrauben, also durch An- bzw. Abschrauben, zu ermöglichen, ist die Außenkappe 6 in einem in Längsrichtung gesehen versetzt zum Kontaktbereich 16 angeordneten Handhabungsbereich 20 an ihrem Außenumfang mit - im Ausführungsbeispiel ― zwei sich in Längsrichtung erstreckenden Griffrillen 22 versehen. Diese bilden die Griffelemente, die für den Bediener den Kraftschluss bei der Schraubbewegung besonders erleichtern.

Der Dichtungsstopfen 8 ist in Fig. 3 im Längsschnitt gezeigt. In seiner Außenkontur ist er hinsichtlich Dimensionierung und Geometriewahl an den Innenkanal 14 im Grundkörper 10 der Außenkappe 6 angepasst, so dass er in diesen einschiebbar ist. In seinem Inneren weist er hingegen seinerseits einen Aufnahmekanal 24 auf, in den eine entsprechend Mündungskontur der jeweiligen Spritze bzw. des medizinischen Behälters einschiebbar ist. Aufgrund der Materialwahl für den Dichtungsstopfen 8, nämlich Gummi oder TPE mit entsprechend weichen, elastischen Eigenschaften, kann der zugeordnete Mündungsbereich der Spritze bzw. des medizinischen Behälters dichtend verschlossen werden.

Das Gewinde-Adapterstück 4 ist in Fig. 4 im Längsschnitt (Fig. 4a) und in Ansicht von unten (Fig. 4b) gezeigt. Es ist im Wesentlichen einstückig aus einem Kunststoff, insbesondere aus Polypropylen (PP), einem Polyolefin, Cyclo-Olefin-Copolymer (COC), Cyclo-Olefine-Polymer (COP) oder Polycarbonat, gefertigt und umfasst einen Außenmantel 26, der mit einem an das Außengewinde 18 der Au-βenkappe 6 angepassten Innengewinde 28 versehen ist. Des Weiteren weist das Gewinde-Adapterstück 4 in einem bodenseitigen Endbereich 30 einen auf einen Endbereich des Spritzenkörpers aufschiebbaren, mit einer Anzahl von umlaufend angeordneten Klemmfedern 32 versehenen Klemmring 34 aufweist.

Zur Bildung der Verschlusskappe 2, die ihrer Bauweise nach grundsätzlich zweikomponentig ausgeführt ist, werden zunächst ihre Komponenten Außenkappe 6 und Dichtungsstopfen 8 zusammengefügt. Insbesondere sollten diese Komponenten für eine erleichterte Handhabung vormontiert sein, so dass bei der eigentlichen Anbringung der Verschlusskappe 2 am Spritzenkörper lediglich ein Bauteil aufgebracht werden muss. Um eine solches stabiles Zusammenfügen der Komponenten zu ermöglichen, ist im Ausführungsbeispiel der in der Außenkappe 6 zur Aufnahme des Dichtungsstopfens 8 vorgesehene Innenkanal 14 mit innenseitig angeformten Rastsegmenten 36 versehen, wie sie insbesondere in der Darstellung im Längsschnitt in der geeigneten Richtung gem. Fig. 2a erkennbar sind. Korrespondierend dazu weist der Dichtungsstopfen 8 eine daran angepasste außenseitig umlaufende Rastkante 38 auf. Diese ist angepasst an den Innenkanal 14 derart dimensioniert und positioniert, dass der Dichtungsstopfen 8 nach seinem Einschieben in den Innenkanal 14 durch Verhakung der genannten Rastelemente rastend im Innenkanal der Außenkappe fixiert wird.

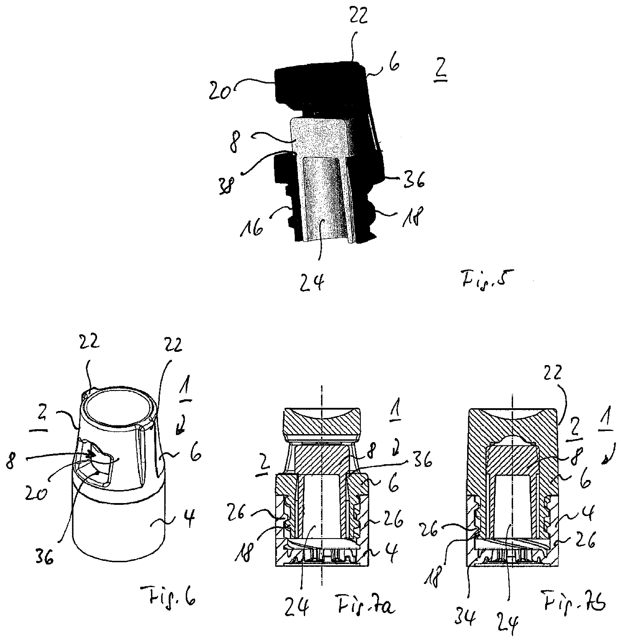

Die durch diese Zusammenfügung entstehende Verschlusskappe 2 ist in Fig. 5 im perspektivischen Schnitt gezeigt. Diese Verschlusskappe 2 ist in dieser Form für ein unmittelbares Anschrauben an den eigentlichen Spritzenkörper vorgesehen und geeignet, sofern dieser nach seiner grundsätzlichen Auslegung ohnehin bereits mit einem geeigneten, mit dem Außengewinde 18 kompatiblen Gewindesystem ausgerüstet ist, beispielsweise um später einen Nadelhalter oder dergleichen am Mündungsbereich des Spritzenkörpers anschrauben zu können.

Alternativ kann die Verschlusskappe 2 auch mit dem Gewinde-Adapterstück 4 zur Bildung des Verschlusssystems 1 kombiniert werden, das beispielsweise auf Spritzenkörper mit einem sich verjüngenden Mündungsbereich aufgeschoben werden kann. Die Fixierung an der Spritze erfolgt dabei über den auf den Endbereich des Spritzenkörpers aufschiebbaren, mit einer Anzahl von umlaufend angeordneten Klemmfedern 32 versehenen Klemmring 34. Zur Anbringung an der Spritze wird bevorzugt und in als eigenständig erfinderisch angesehener Ausgestaltung auch hier das Verschlusssystem 1 vor der eigentlichen Anbringung an der Spritze vormontiert, indem die Verschlusskappe 2 mit dem Außengewinde 18 in das Innengewinde 28 des Gewinde-Adapterstücks 4 eingeschraubt wird. Ein solchermaßen vormontiertes Verschlusssystem 1 ist in Fig. 6 in perspektivischer Ansicht und in Fig. 7 in zwei Längsschnitt-Darstellungen gezeigt.

Die erfindungsgemäße Verschlusskappe 2 stellt einerseits die erwünschte hohe Dichtwirkung durch das in dieser Hinsicht geeignet ausgelegte Dichtelement 8 und andererseits die erwünschte hohe mechanische Stabilität und Belastbarkeit durch die schützende Außenkappe 6 bereit.

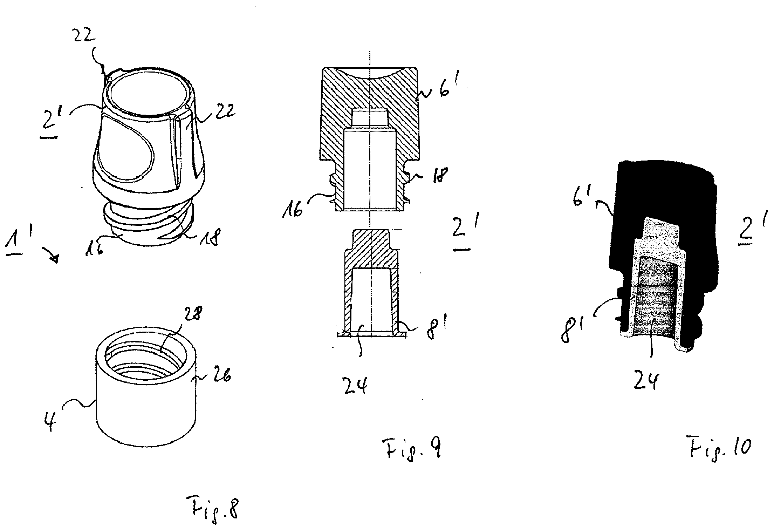

Ein alternatives Verschlusssystem 1' mit einer Verschlusskappe 2' in ebenfalls als eigenständig erfinderisch angesehener Ausführung ist in Fig. 8 in Explosionsdarstellung gezeigt. Es umfasst als Hauptkomponenten die alternative Verschlusskappe 2' sowie ebenfalls das zugeordnete Gewinde-Adapterstück 4. Die Verschlusskappe 2' ist auch in dieser Variante zweikomponentig ausgeführt, so dass auch in der Art einer funktionalen Trennung mehrere eigentlich konkurrierende Auslegungsziele erreicht werden können. In dieser alternativen, als eigenständig erfinderisch angesehen Ausgestaltung ist die Verschlusskappe 2' aber durch ein Zwei-Komponenten-Spritzgussverfahren hergestellt, bei dem die beiden Komponenten von vornherein gemeinsam miteinander produziert werden. Die Komponenten der Verschlusskappe 2' sind in Fig. 9 im Längsschnitt und der Übersichtlichkeit halber in Explosionsdarstellung gezeigt, obwohl sie herstellungsbedingt nicht separat vorliegen. Fig.10 zeigt hingegen die vollständige, im Zwei-Komponenten-Spritzgussverfahren hergestellte Verschlusskappe 2' im perspektivischen Schnitt.

Auch die Verschlusskappe 2' gem. Fig. 10 ist in dieser Form für ein unmittelbares Anschrauben an den eigentlichen Spritzenkörper vorgesehen und geeignet, sofern dieser nach seiner grundsätzlichen Auslegung ohnehin bereits mit einem geeigneten, mit dem Außengewinde 18 kompatiblen Gewindesystem ausgerüstet ist, beispielsweise um später einen Nadelhalter oder dergleichen am Mündungsbereich des Spritzenkörpers anschrauben zu können.

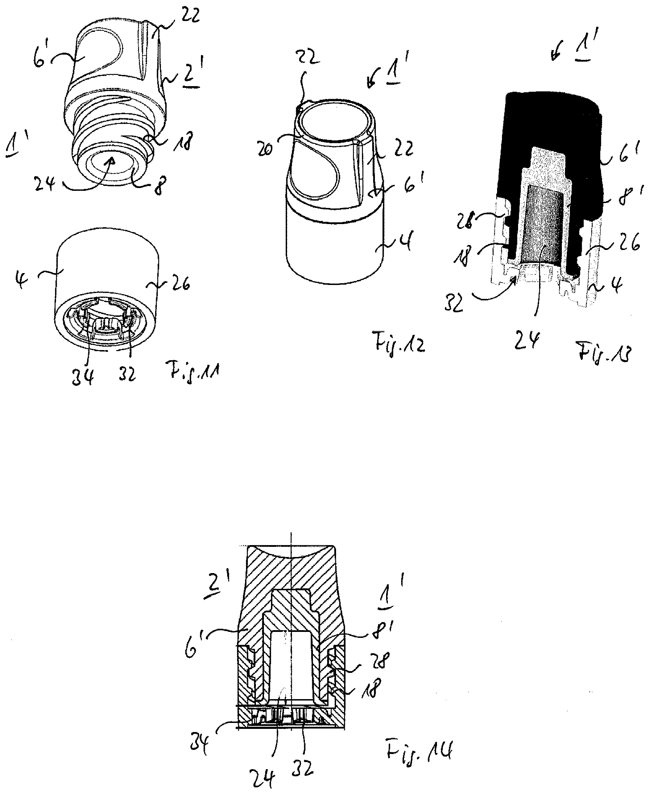

Alternativ kann aber auch die Verschlusskappe 2' analog zur vorstehend beschriebenen Variante mit dem Gewinde-Adapterstück 4 zur Bildung des Verschlusssystems 1 kombiniert werden, das beispielsweise auf Spritzenkörper mit einem sich verjüngenden Mündungsbereich aufgeschoben werden kann. Zur Anbringung an der Spritze wird bevorzugt und in als eigenständig erfinderisch angesehener Ausgestaltung auch hier das Verschlusssystem 1' vor der eigentlichen Anbringung an der Spritze vormontiert, indem die Verschlusskappe 2' mit dem Außengewinde 18 in das Innengewinde 28 des Gewinde-Adapterstücks 4 eingeschraubt wird, wie durch die Explosionsdarstellung in Fig. 11 angedeutet. Das solchermaßen vormontierte Verschlusssystem 1' ist in Fig. 12 in perspektivischer Ansicht, in Fig. 13 im perspektivischen Schnitt und in Fig. 14 im Längsschnitt gezeigt.

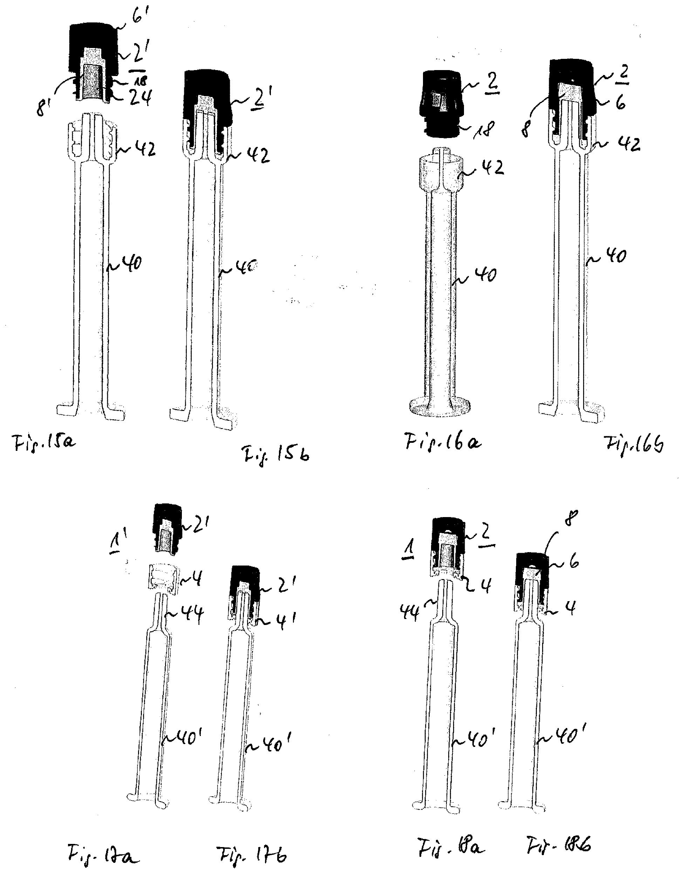

In den Figuren 15 bis 18 ist jeweils ein Spritzenkörper 40, 40' in einer Ausführung mit angeformtem Gewindesystem 42 (Spritzenkörper 40) bzw. in einer Ausführung ohne derartiges Gewindesystem und stattdessen mit einem sich verjüngenden Mündungsbereich 44 versehen (Spritzenkörper 40') gezeigt, und zwar in der Darstellung links jeweils vor und rechts nach der Anbringung des jeweiligen Verschlusselements.

Beim mit dem angeformten Gewindesystem 42 versehenen Spritzenkörper 40 kann es sich insbesondere um einen als Standardprodukt erhältlichen und weit verbreiteten vorbefüllbaren vergleichsweise kleinvolumigen Spritzenkörper 40 (Füllvolumen beispielsweise 0,5 ml) mit angeformtem Luer-Gewinde, beispielsweise erhältlich als "0,5 ml integrierte Polymer-Luer-Gewinde-Spritze", handeln. Das Gewindesystem 42 kann dabei beispielsweise vorgesehen sein, um später einen Nadelhalter oder dergleichen am Mündungsbereich des Spritzenkörpers 40 anschrauben zu können. Die vorstehend beschriebenen Verschlusskappen 2, 2' mit ihren bevorzugten Ausführungsformen sind dem Grunde nach für ein Anschrauben an den eigentlichen Spritzenkörper 40 vorgesehen und dementsprechend bei der Ausgestaltung ihres Außengewindes 18 an das Gewindesystem 42 angepasst. Sie können somit unmittelbar mit einem solchen Typ von Spritzenkörper 40 verwendet werden.

Alternativ und unter ergänzendem Rückgriff auf das Gewinde-Adapterstück 4 sind die Verschlusskappen 2, 2' aber auch bei dem alternativen, auch als "Standard-Spritze" bezeichneten Typus von Spritzenkörper 40' mit sich verjüngendem Mündungsbereich 44 verwendbar. Ein solcher vorbefüllbarer Spritzenkörper 40' kommt weit verbreitet beispielsweise als Standard-Spritze mit einem Füllvolumen von 1 ml zum Einsatz. Dazu kann das jeweilige Verschlusssystem 1,1' unter Nutzung des Klemmrings 34 auf den Mündungsbereich 44 aufgeschoben und dort verklemmt werden.

### Bezugszeichenliste

- 1,1': Verschlusssystem
- 2, 2': Verschlusskappe
- 4: Gewinde-Adapterstück
- 6, 6': Außenkappe
- 8, 8': Dichtungsstopfen
- 10: Grundkörper
- 12: Längsachse
- 14: Innenkanal
- 16: Kontaktbereich
- 18: Außengewinde
- 20: Handhabungsbereich
- 22: Griffrille
- 24: Aufnahmekanal
- 26: Außenmantel
- 28: Innengewinde
- 30: Endbereich
- 32: Klemmfeder
- 34: Klemmring
- 36: Rastsegment
- 38: Rastkante
- 40, 40': Spritzenkörper
- 42: Gewindesystem
- 44: Mündungsbereich

## Patentansprüche

1. Verschlusskappe (2, 2') für eine mit einem Medikament oder medizinischen Wirkstoff vorbefüllte Spritze (40, 40'), mit einer starren, in einem Kontaktbereich (16) mit einem Außengewinde (18) versehenen Außenkappe (6, 6'), die einen sich in einer Längsrichtung erstreckenden Innenkanal (14) aufweist, in dem ein Dichtungsstopfen (8, 8') aus einem im Vergleich zur Au-βenkappe (6, 6') weicheren und besser verformbaren Material, vorzugsweise aus Gummi oder TPE, angeordnet ist.

2. Verschlusskappe (2, 2') nach Anspruch 1, deren Außenkappe (6, 6') aus einem Kunststoff, vorzugsweise aus Polypropylen (PP), einem Polyolefin, Cyclo-Olefin-Copolymer (COC), Cyclo-Olefine-Polymer (COP) oder Polycarbonat gefertigt ist.

3. Verschlusskappe (2, 2') nach Anspruch 1 oder 2, deren Außenkappe (6, 6') in einem in Längsrichtung gesehen versetzt zum Kontaktbereich (16) angeordneten Handhabungsbereich (20) an ihrem Außenumfang mit einer Anzahl von, vorzugsweise mit zwei, sich in Längsrichtung erstreckenden Griffrillen (22) versehen ist.

4. Verschlusskappe (2, 2') nach einem der Ansprüche 1 bis 3, deren Außengewinde (18) als Luer-Gewinde ausgeführt ist.

5. Verschlusskappe (2) nach einem der Ansprüche 1 bis 4, bei der der in der Außenkappe (6) vorgesehene Innenkanal (14) mit an seinem Innenumfang angeordneten Rastsegmenten (36) versehen ist, und bei der der Dichtungsstopfen (8) eine daran angepasste außenseitig umlaufende, derart dimensionierte Rastkante (38) aufweist, dass der Dichtungsstopfen (8) nach seinem Einschieben in den Innenkanal (14) rastend fixiert wird.

6. Verschlusskappe (2') nach einem der Ansprüche 1 bis 4, erhalten durch Herstellung im Zwei-Komponenten-Spritzgussverfahren.

7. Verschlusssystem (1, 1') für eine mit einem Medikament oder medizinischen Wirkstoff vorbefüllte Spritze (40'), mit einer Verschlusskappe (2, 2') nach einem der Ansprüche 1 bis 6, und mit einem Gewinde-Adapterstück (4), das in einem Außenmantel (26) mit einem an das Außengewinde (18) der Außenkappe (6, 6') angepassten Innengewinde (28) versehen ist, und das einen auf einen sich verjüngenden Mündungsbereich (44) des Spritzenkörpers (40') aufschiebbaren, mit einer Anzahl von umlaufend angeordneten Klemmfedern (32) versehenen Klemmring (34) aufweist.

8. Verschlusssystem (1, 1') nach Anspruch 7, dessen Gewinde-Adapterstück (4) aus einem Kunststoff, vorzugsweise aus Polypropylen (PP), einem Polyolefin, Cyclo-Olefin-Copolymer (COC), Cyclo-Olefine-Polymer (COP) oder Polycarbonat gefertigt ist.
